# EUROPEAN PATENT SPECIFICATION

(11) **EP 1 371 322 B1**
(45) Date of publication and mention of the grant of the patent: **08.08.2007**
(21) Application number: 02425386.6
(22) Date of filing: 14.06.2002
(51) Int. Cl.: A61B 3/107, A61B 3/11

(54) **Corneal topograph with integrated pupil test device**
Kornea-Topograph mit integrierter Pupillentest-Vorrichtung
Topographe pour la cornée avec testeur de pupille intégré

(43) Date of publication of application: 17.12.2003
(73) Proprietor: C.S.O. SRL, 50010 Badia a Settimo, Florence (IT)
(72) Inventor: Mura, Sergio, I-50018 Scandicci (Florence) (IT)
(74) Representative: Bardini, Marco Luigi

(56) References cited:
- EP-A2- 1 057 446
- US-B1- 6 224 213
- BOXER WACHLER B S ET AL: "Agreement and repeatability of pupillometry using videokeratography and infrared devices." JOURNAL OF CATARACT AND REFRACTIVE SURGERY. UNITED STATES JAN 2000, vol. 26, no. 1, January 2000 (2000-01), pages 35-40, XP002223443 ISSN: 0886-3350

## Description

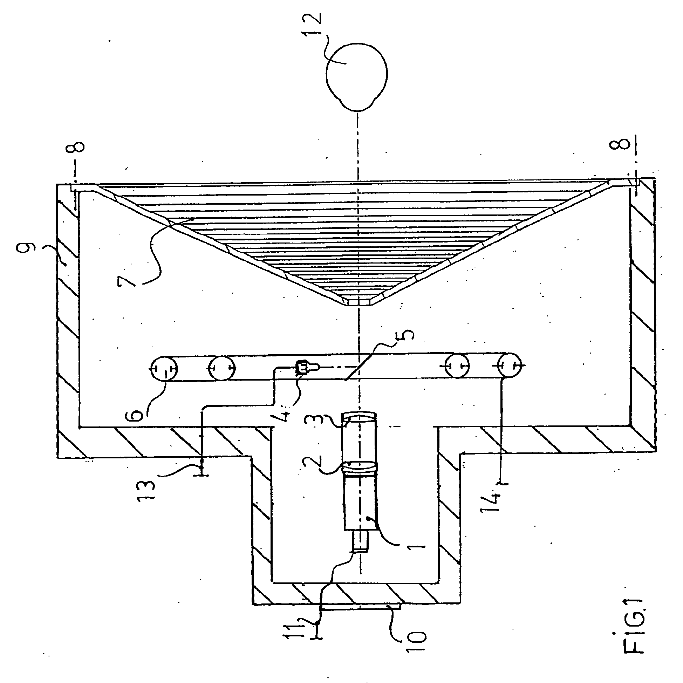

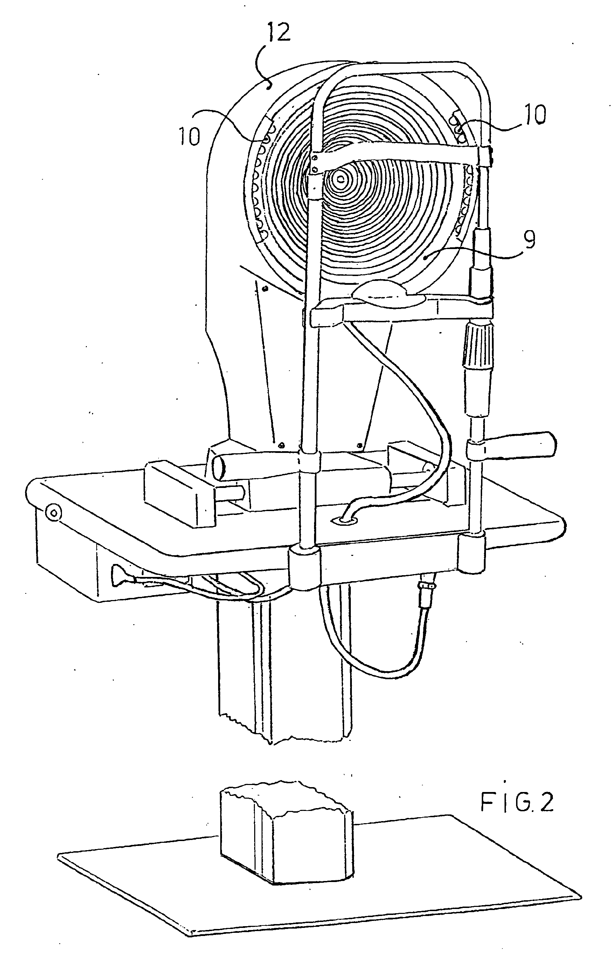

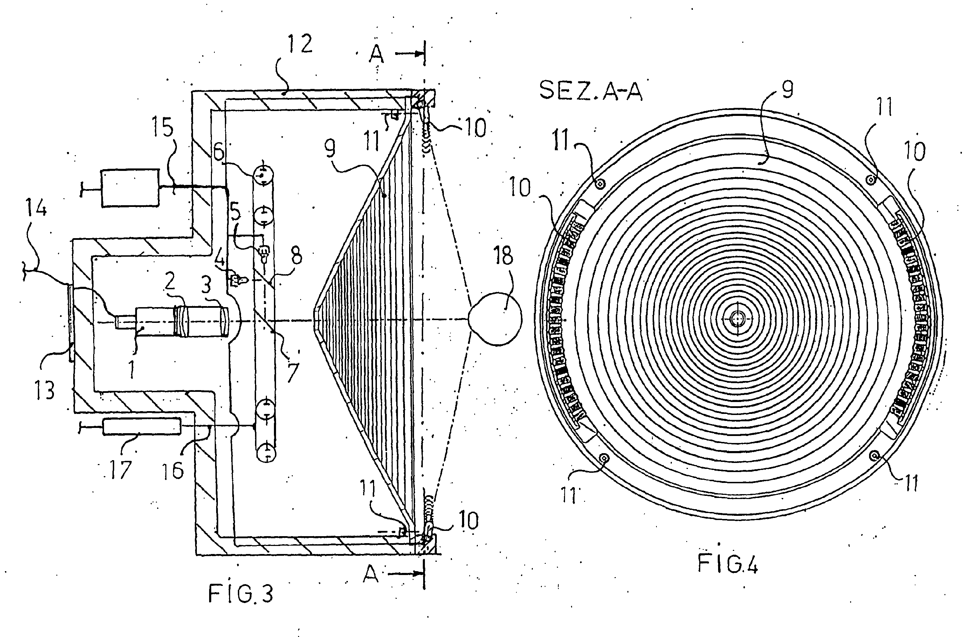

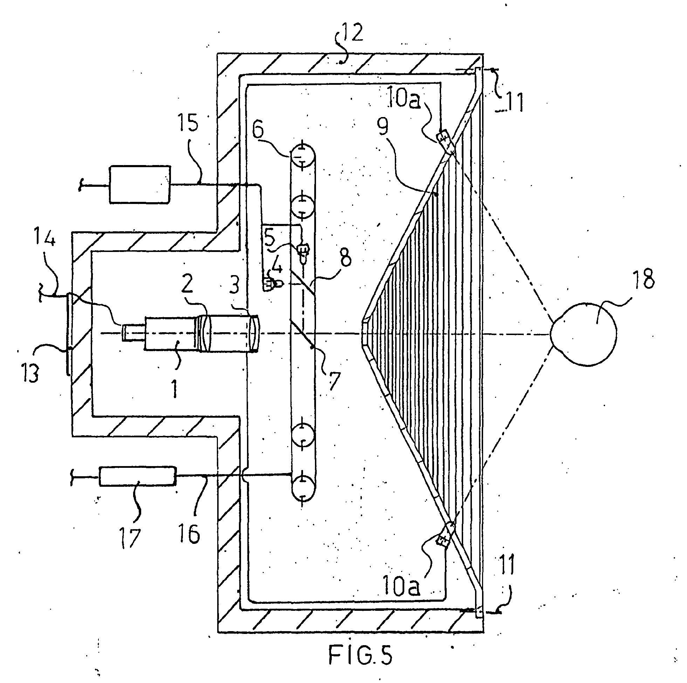

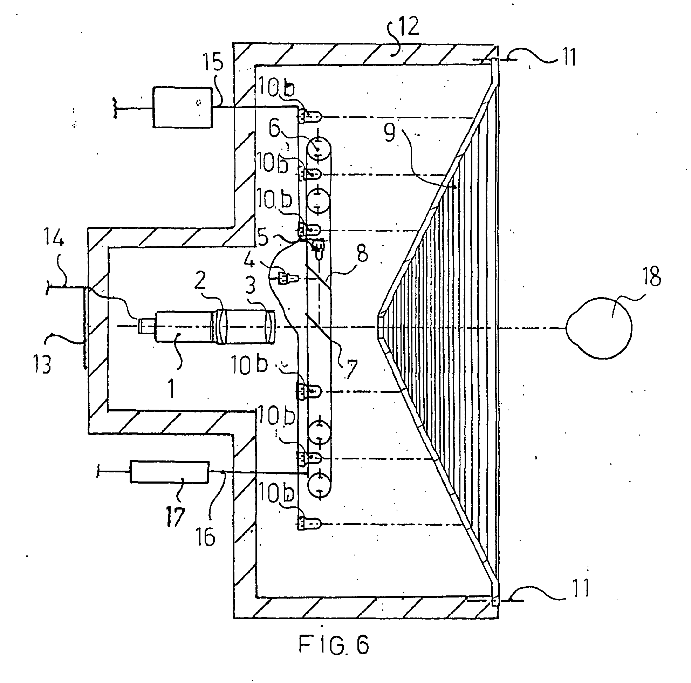

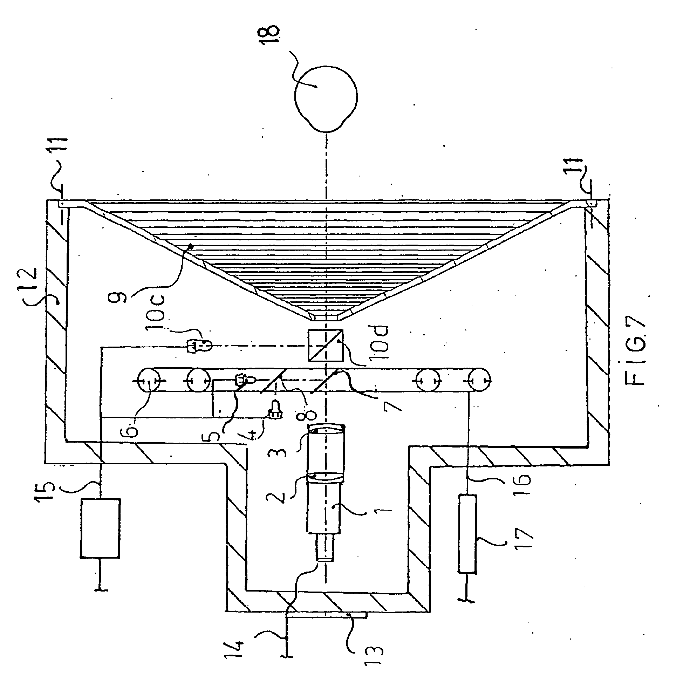

It is known that the test of the cornea or of the pupil is basically in the ophthalmology. Infact the acquisited data to this test are particular important both to the diagnostic point of view than to have extremely precise data before a refractive surgery operation. According to the prior art the cornea examination is carried out by a device known like corneal topography. Said known device consists (as illustrated in figure 1) of a videocamera 1 for image taking coming from the lens 2 and 3. Then said device provides a fixing LED (light-emitting diode) 4 that goes to be reflected onto the silver-plated wall of a glass semi-silver-plated plate 5. Then a neon glow lamp 6 is provided that, during its ignition, lights up the Placido disk or keratography 7 fixed, by means of fasteners 8, to the frame 9 of the same device. Outwardly to said frame a small monitor 10 is placed connected to the videocamera 1 by means of the connection wire 11 to permit the prompt visualisation of what visualisated. Said wire 11 for the acquisition then to be connected with a second monitor and with a computer. During its functioning the patient's eye 12 is placed in examining position whereas the wires 13 and 14 permit the electrical connections of the LED 4 and of the neon glow lamp 6. During this functioning the plate 5, lit up by the LED 4, reflects on its outside part what the patient observes, that is the fixing centre. In this situation onto the cornea is formed what is known like the first Purchinjè image. Said image, taken to the optic of the system, is filmed to the videocamera that sends the same to the small monitor 10 by means of the wire 11. Said digital images then to be sent to a computer with a system software which permits the elaboration of images. An apparatus according to the prior art is shown in US 6224213. The invented device, in comparison with the cited device which actuated a normal solution in use between the bigger part of the manufacturers, permits to actuate with only one device also the pupil test and, particular of big importance, it is able to point out the pupil centre on the light alteration, changeable from patient to patient. Moreover the same device is able to determine, without image interruption, the pupil morphology, this data changeable and peculiar to each patient. Practically the invented device is able to film the pupil variations with the different light conditions and, elaborating the area of the same pupil, is able to set for each light condition the pupil centre for each examinated eye. On this base the same device contemporary gives the fixing centre and the pupil centre. The acquisition of said points, distinct and separated, is of foundamental importance for the refractive surgery operation treated with laser. It is known that in these operations is generally used an eximer laser, instrument extremely precise that, availing itself of two data sources, gives better operation results in comparison with those obtained considering for the operation only the fixing point and no knowing, in this situation, in continuos the area variability of the pupil and consequently its centre. Infact the pupil areas change from a maximumvalue, obtained without light, to a minimum value obtained with the highest light of the lamp presents inside the device, always considering the keratometric axis such as the patient alwasys observes the fixing point. Obviously, by means of the videocamera filming, it is possible to know all the intermediate positions that are memorized so to be using for any computer elaboration. The invented corneal topography consists of a videocamera 1 in sequence with the optical group having the lens 2 and 3. Then a fixing LED 4 and an infrared fixing LED 5 with a neon glow lamp 6 with two beam splitter plates 7 and 8 are provided. In the outside part is then provided a Placido disk or keratography 9 having laterally positioned infrared LED 10. Said LED 10 placed in this position to be able to give grazing light for the pupil test. Said LED 10 and the fixing LED 5 are infrared so to not interfere with the light sources to be used for the cornea test, infact, for this test frequence in the visible light field are used.The Pla cido disk 9 is mounted , by means of bolts 11, to the device frame 12. Said body 12 outside is connected with a small monitor 13 connected to the videocamera 1 by wire 14. This said wire 14 permits to send the signal also to a second outside monitor connected with a computer with a system software able to elaborated the images. The various LED connected each other by wires 15 with network connection. Also the neon glow lamp is connected by wire 16 using, for the luminous intensity variability, a potentiometer 17. During the use of the topography, the patient eye 18 to be tested is therefore contemporary subjected to the cornea test and to the pupil test. Within the art of topography the infrared LED 10 are to be provided also in different position on the base of the use necessity. In an embodiment which does not fall under the scope of claim 1 the infrared LED 10a are placed inside the Placido disk 9. In a second embodiment which does not fall under the scope of claim 1 a set of infrared LED 10b are provided in parallel position in comparison with the lamp 6. In a third embodiment which does not fall under the scope of claim 1 only one infrared LED 10c is provided with beam splitter plate 19 having the same function of the previous applications. The device is illustrated in the different embodiments and in indicative way not limiting in the drawings of sheets 1, 2, 3, 4, 5 and 6. In sheet 1 the fig. 1 is transversal section view of the corneal topography known from the previous art. The sheets 2 and 3 show the invented topography in the preferred embodiment. In particular in sheet 2 fig. 2 is total perspective view of the topography. In sheet 3 fig. 3 is longitudinal section view passing to the Placido disk 9 centre. Fig. 4 is transversal section view. In sheet 4 fig. 5 is longitudinal section view of the embodiment with LED 10a. In sheet 5 fig. 6 is longitudinal section view of the embodiment with LED 10b. In sheet 6 fig. 7 is longitudinal sectio of the embodiment with LED 10c.

## Claims

1. Corneal topography with integrated pupil test device comprising a videocamera (1) in sequence with an optical group with lens (2 and 3) and with an outside Placido Disk (1), a neon glow lamp (6) for lighting up the Placido Disk (1) for the topography of the cornea, a fixing LED (4) and an infrared fixing LED (5) associated with respective beam splitter plates (7, 8), **characterized in that** an array of additional infrared LEDs (10) is provided for the pupil test, placed laterally onto the Placido disk to give grazing light for the pupil test.

## Patentansprüche

1. Kornea-Topograph mit integrierter Pupillentest-Vorrichtung, enthaltend eine Videokamera (1) in einer Abfolge mit einer optischen Gruppe mit einer Linse (2 und 3) und mit einer äußeren Placido-Disk (1), einer Neonglühlampe (6) zum Erhellen der Placido-Disk (1) für die Topographie der Kornea, eine Fixier-LED (4) und eine infrarote Fixier-LED (5) in Verbindung mit jeweiligen Stahlteilerplatten (7, 8), **dadurch gekennzeichnet, dass** eine Anordnung von zusätzlichen Infrarot-LEDs (10) für den Pupillentest vorgesehen ist, angeordnet seitlich an der Plazido-Disk, um ein Streiflicht für den Pupillentest zu ergeben

## Revendications

1. Topographe pour la cornée avec testeur de pupille intégré comportant une caméra vidéo (1) en série avec un bloc optique de lentilles (2 et 3) et avec un Disque de Placido extérieur (9), une lampe au néon (6) pour illuminer le Disque de Placido (9) pour la topographie de la cornée, une LED de pointage (4) et une LED infrarouge de pointage (5) respectivement associées à des lames séparatrices de faisceaux (7, 8), **caractérisé en ce qu'**une matrice de LEDs infrarouges additionnelles (10) est prévue pour le test de pupille, située latéralement sur le Disque de Placido pour donner une lumière effleurante pour le test de pupille.
